# EUROPEAN PATENT APPLICATION

(11) **EP 4 480 378 A1**
(43) Date of publication of application: **25.12.2024**
(21) Application number: 23831668.1
(22) Date of filing: 23.03.2023
(51) Int. Cl.: A47L 23/20, A47B 61/04, A61L 2/07, F26B 21/08, F26B 21/00, F26B 21/06

(54) **SHOE CARE APPARATUS**

(30) Priority: 01.07.2022 KR 20220081451
(71) Applicant: LG Electronics Inc., Yeongdeungpo-gu Seoul 07336 (KR)
(72) Inventor: KIM, Chang Kyu, Seoul 08592 (KR); BAN, Seok Hyun, Seoul 08592 (KR); PARK, Hye Yong, Seoul 08592 (KR)
(74) Representative: Schott, Jakob Valentin
(86) International application number: PCT/KR2023/003827
(87) International publication number: WO 2024/005301

(57) **Abstract**

Provided is a shoe care device in which shoes are processed by circulating air flow. The shoe care device according to an aspect of the present disclosure includes: an inner cabinet having an accommodation space configured to accommodate shoes; a connection path configured to provide a flow path through which air from the accommodation space is introduced and then discharged back into the accommodation space; a blowing part disposed in the connection path and configured to blow air; a dehumidifying part disposed in the connection path and configured to dehumidify the air; a controller configured to control a drying stroke in which air moves along the connection path and is dehumidified to dry the accommodation space; a first sensor disposed in a portion of the connection path through which air is introduced into the dehumidifying part to measure a temperature of the air; and a second sensor disposed in a portion of the connection path through which the air is discharged from the dehumidifying part to measure a temperature of the air. The controller is configured to control the drying stroke to be performed until a state in which completion of processing of the shoes is estimated through a difference of the temperatures of air measured by the first sensor and the second sensor during the drying stroke.

## Description

### TECHNICAL FIELD

The present invention relates to a shoes care device, and more particularly, to a shoes care device in which shoes are processed by air circulation.

### BACKGROUND

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device 1, which removes germs and odors by performing a predetermined treatment on the shoes so that a user can comfortably wear the shoes all the times.

For the shoes care device, Korean Patent No. 1037245 (hereinafter, referred to as "Prior Document 1") discloses "Apparatus for sterilization disposal of shoes", which includes a main body, an ultraviolet emission module, and a deodorization module.

According to Prior Document 1 above, the shoes are disposed in a sterilization chamber of the main body, and the ultraviolet emission module is driven to remove germs and odors of the shoes. Furthermore, the air in the sterilization chamber is inhaled into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Document 1 above, the air from which moisture has been removed by a deodorization module including zeolite and activated carbon can be discharged to the outside of the apparatus for sterilization disposal of shoes.

However, in Prior Document 1 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Document 2") discloses "The shoes cabinet for sanitation", which includes a main body, a far infrared radiation part, a circulation fan, an air circulation passage, and a sanitary filter portion.

According to Prior Document 2 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Document 2 above, the air is circulated by a circulating fan in a shoe closet, and the sanitary filter portion is disposed on a circulation path of the air to remove germs and odors in the air.

However, since Prior Document 2 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the use of the shoes care device and the regeneration of the dehumidifying material can be effectively removed or managed, whether moisture is left in an unintended area during use of the shoes care device and the regeneration of the dehumidifying, whether components are properly disposed in a limited space, and where the devices provides excellent use convenience.

In addition, the reduction of manufacturing costs, the convenience of manufacturing and assembling each component, and the convenience of maintenance needs to be considered in the development of the shoes care device.

### DISCLOSURE

### TECHNICAL PROBLEM

An object to be achieved by the present invention is to solve the aforementioned problems caused by the shoes care device which treats shoes by air circulation.

Specifically, an object to be achieved by the present invention is to provide a shoes care device which can ensure proper performance for shoes treatment at all times by performing dehumidification and deodorization on the shoes using a dehumidifying material to refresh the shoes and regenerating the used dehumidifying material.

An object to be achieved by the present invention is to provide a shoes care device which prevents air used for dehumidification and deodorization of shoes from being exposed to a user by an air circulation structure capable of dehumidifying the air inside an inner cabinet where the shoes are placed by using a dehumidifying material and supplying the dehumidified air again to the inner cabinet.

An object to be achieved by the present invention is to provide a shoe care device that can automatically terminate operation without separate manipulation by a user by applying a structure that is capable of estimating that processing of shoes has been completed.

The technical objects of the present disclosure are not restricted to those set forth Here, and other unmentioned technical objects will be clearly understood by one of ordinary skill in the art to which the present disclosure pertains by referencing the detailed description of the present disclosure given below.

### TECHNICAL SOLUTION

In order to achieve the object or another object, a shoes care device thereof according to an aspect of the present invention are configured so that shoes are dehumidified and deodorized by a dehumidifying part, and the used dehumidifying part is regenerated. Specifically, the dehumidifying part is disposed in a module chamber to collect moisture and bacteria in blown air, and the dehumidifying part may be heated and regenerated in the module chamber.

Further, the shoes care device according to an aspect of the present invention are configured so that the air used for dehumidifying and deodorizing the shoes has a circulation air current structure. Specifically, a connection path in which air is circulated is configured to be formed between a outlet and a nozzle each disposed inside an inner cabinet.

Further, the shoe care device according to an aspect of the present invention is configured to perform a stroke until a state is achieved in which completion of processing of the shoes is able to be estimated without a separate operation by a user. Specifically, the shoe care device is configured to perform a drying stroke until the state is achieved in which completion of processing of the shoes is able to be estimated through a difference of the temperatures of air introduced into the dehumidifying part and air discharged from the dehumidifying part.

Further, the shoe care device according to an aspect of the present invention may terminate the drying stroke when the temperature difference is at or below a set value or lower.

Further, the shoe care device according to an aspect of the present invention may perform the regeneration stroke and the drying stroke at least once in the operating mode.

Further, the shoe care device according to an aspect of the present invention may terminate the drying stroke when the temperature difference is continuously at or below the set value a first set number of times or more.

Further, when the shoe care device according to an aspect of the present invention may terminate the drying stroke when the temperature difference as much as a second set number of times.

Further, when the drying stroke is re-performed after a temporary stop, the shoe care device according to an aspect of the present invention may perform a stroke continuously to the stroke performed before the temporary stop without initializing the stroke.

Further, the shoe care device according to an aspect of the present invention performs a regeneration stroke and a drying stroke in one cycle, and in this cycle, the regeneration stroke may be performed prior to the drying stroke.

Further, the shoe care device according to an aspect of the present disclosure may perform a steam stroke in an operating mode.

Further, the shoe care device according to an aspect of the present disclosure may perform the steam stroke between the first regeneration stroke and the first drying stroke.

Further, the shoe care device according to an aspect of the present invention may include a first module chamber, a second module chamber, and a third module chamber that are provided at different positions, respectively, in a plan view and accommodate a blowing part, a heating part, and a dehumidifying part, respectively.

Further, the shoe care device according to an aspect of the present invention may be configured to allow air to sequentially move through the first module chamber, the second module chamber, and the third module chamber.

Further, the shoe care device according to an aspect of the present invention may be configured to measure the temperature of air introduced into the dehumidifying part in the second module chamber and measure the temperature of air discharged from the dehumidifying part in the third module chamber.

The technical objects of the present disclosure are not restricted to those set forth Here, and other unmentioned technical objects will be clearly understood by one of ordinary skill in the art to which the present disclosure pertains by referencing the detailed description of the present disclosure given below.

### BRIEF DESCRIPTION OF THE DRAWINGS

FIG. 1a is a perspective view illustrating a shoes care device according to one embodiment of the present invention.
FIG. 1b is a perspective view of the shoes care device of FIG. 1a, when viewed from another direction, illustrating a state in which a door is opened.
FIG. 2a is a perspective view illustrating a state in which a part of the door and an outer cabinet are removed from the shoes care device of FIG. 1b.
FIG. 2b is a perspective view illustrating a state in which the shoes care device illustrated in FIG. 2a is viewed from another direction.
FIG. 3 is a front view illustrating a state in which a door is removed from the shoes care device illustrated in FIG. 1b. FIG. 3 illustrates shoes accommodated in an inner cabinet together.
FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.
FIG. 5 is a perspective view illustrating a state in which the door, the outer cabinet, and the inner cabinet are removed from the shoes care device according to one embodiment of the present invention.
FIG. 6 is a perspective view illustrating a part of a machine room of the shoes care device illustrated in FIG. 5.
FIG. 7a is a view illustrating a steam valve according to one embodiment of the present invention, and illustrating a connection relationship considering the movement of steam.
FIG. 7b is an exploded perspective view illustrating the steam valve illustrated in FIG. 7a.
FIG. 8a is a cross-sectional view taken along line D-D' of the shoes care device illustrated in FIG. 3. FIG. 8a illustrates a state in which the door is included in the shoes care device, and does not illustrate the shoes.
FIG. 8b is a view illustrating a state in which a main shelf is removed from the shoes care device illustrated in FIG. 8a.
FIG. 9 is a cross-sectional view taken along line E-E' of the shoes care device illustrated in FIG. 3. FIG. 9 illustrates a state in which the door is included in the shoes care device.
FIG. 10a is an exploded perspective view illustrating a drying module in the shoes care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoes care device according to one embodiment of the present invention.
FIG. 12 is a diagram illustrating a stroke progress depending on the type of operation signal in a control method of the shoes care device according to an embodiment of the present invention.
FIG. 13 is a diagram illustrating the stroke progress after a current mode is switched to an operation mode in the control method of the shoes care device according to an embodiment of the present invention.
FIG. 14 is a diagram exemplarily illustrating a first performance mode in the control method of the shoes care device according to an embodiment of the present invention.
FIG. 15 is a diagram exemplarily illustrating a second performance mode in the control method of the shoes care device according to an embodiment of the present invention.
FIG. 16 is a diagram exemplarily illustrating a third performance mode in the control method of the shoes care device according to an embodiment of the present invention.
FIG. 17 is a diagram illustrating a temperature change of air passing through a dehumidifying part in the shoes care device according to an embodiment of the present invention.
FIG. 18 is a diagram illustrating a humidity change depending on a temperature difference of the air passing through the dehumidifying part in the shoes care device according to an embodiment of the present invention.

### DETAILED DESCRIPTION

Hereinafter, embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A first direction (X direction), a second direction (Y direction) (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

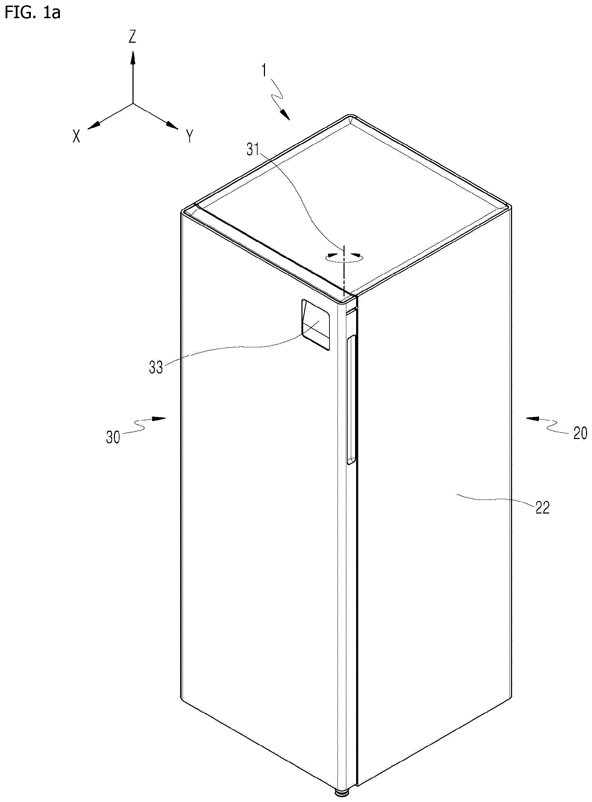

FIG. 1a is a perspective view illustrating a shoes care device according to one embodiment of the present invention.

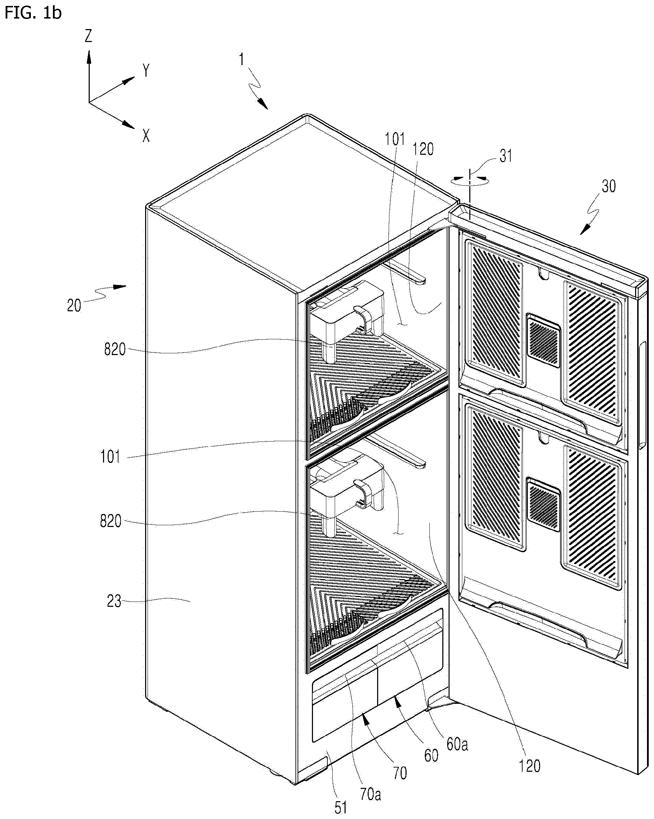

FIG. 1b is a perspective view of the shoes care device of FIG. 1a, when viewed from another direction, illustrating a state in which a door is opened.

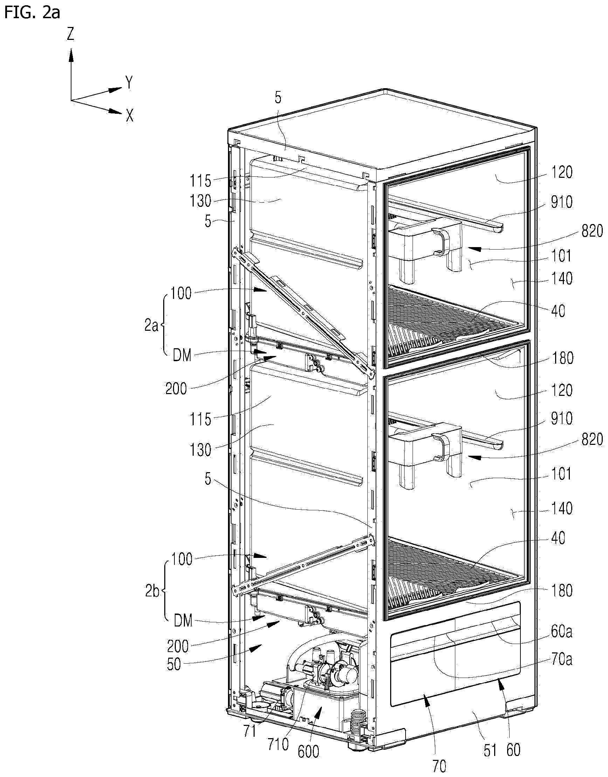

FIG. 2a is a perspective view illustrating a state in which a part of the door and an outer cabinet are removed from the shoes care device of FIG. 1b.

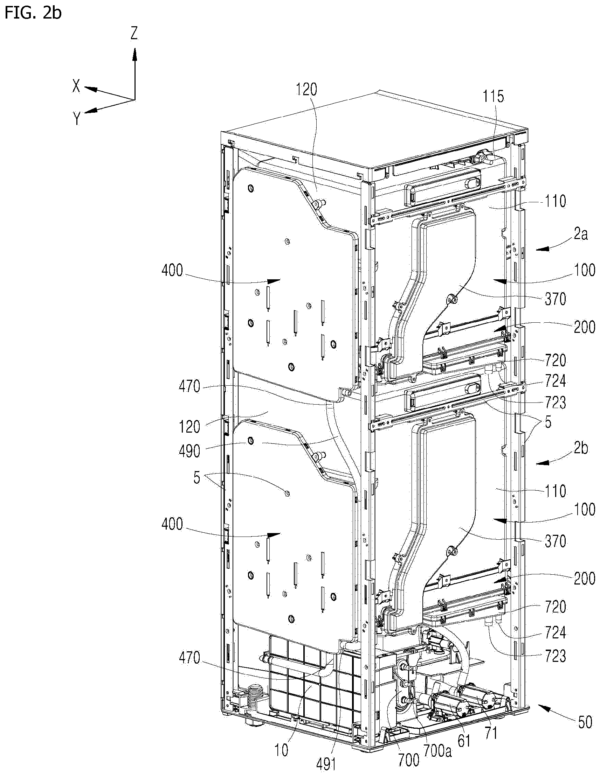

FIG. 2b is a perspective view illustrating a state in which the shoes care device illustrated in FIG. 2a is viewed from another direction.

A shoes care device 1 according to one embodiment of the present invention may include an outer cabinet 20, a door 30, an inner cabinet 100, and a machine room 50. The shoes care device 1 may include a main frame 5.

The outer cabinet 20 and the door 30 may form an overall appearance of the shoes care device 1. The outside of the shoes care device 1 may be formed in a hexahedral shape. That is, while the outer cabinet 20 and the door 30 are coupled to each other and the door 30 is closed, the appearance of the shoes care device 1 may be formed in a hexahedral shape. However, the shoes care device 1 according to one embodiment of the present invention is not limited to such a shape and may have various three-dimensional shapes.

When the door 30 forms the front of the shoes care device 1, the outer cabinet 20 may form an upper side surface, a left-side surface, a right-side surface, a rear surface, and bottom surfaces of the shoes care device 1.

The main frame 5 may form an overall framework of the shoes care device 1. The main frame 5 may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoes care device 1. The outer rear plate 21 may form an entire outer rear surface of the shoes care device 1.

The first outer side plate 22 and the second outer side plate 23 form vertically erected wall surfaces, respectively, and form opposing wall surfaces facing each other.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoes care device 1, the outer cabinet 20 may form a wall separating the machine room 50 from the outside.

The door 30 is configured to open and close the inside of the shoes care device 1. The door 30 may form any one surface of the shoes care device 1. The door 30 may form a left side or a right side of the shoes care device 1, or may form a front side of the shoes care device 1.

In the shoes care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

In one embodiment, the shoes care device 1 may include one door 30. In another embodiment, the shoes care device 1 may include two or more doors.

When two doors are provided in the shoes care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoes care device 1 to the front.

Hereinafter, except for a particularly limited case, it will be described that the door 30 is formed in a front side of the shoes care device 1. That is, a surface on which the door 30 is formed in the shoes care device 1 is described as a front surface of the shoes care device 1.

Furthermore, hereinafter, except for a particularly limited case, it will be described that the first direction (X direction is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoes care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 115 may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100 (see FIGS. 53 and 54).

However, the shoes care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoes care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoes care device 1 may be accommodated, and, in this case, the components that are accommodated in the machine room 50 may be fixed to a main frame 5 or to the inner cabinet 100 or the outer cabinet 20.

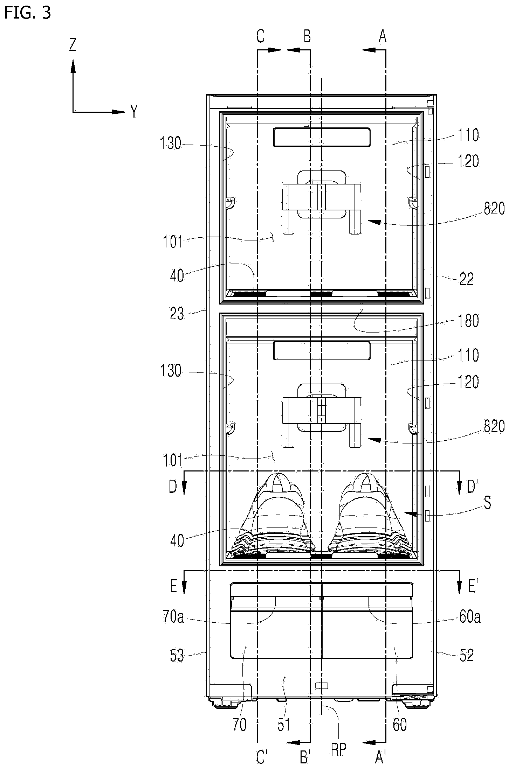

FIG. 3 is a front view illustrating a state in which a door is removed from the shoes care device illustrated in FIG. 1b. FIG. 3 illustrates shoes accommodated in an inner cabinet together.

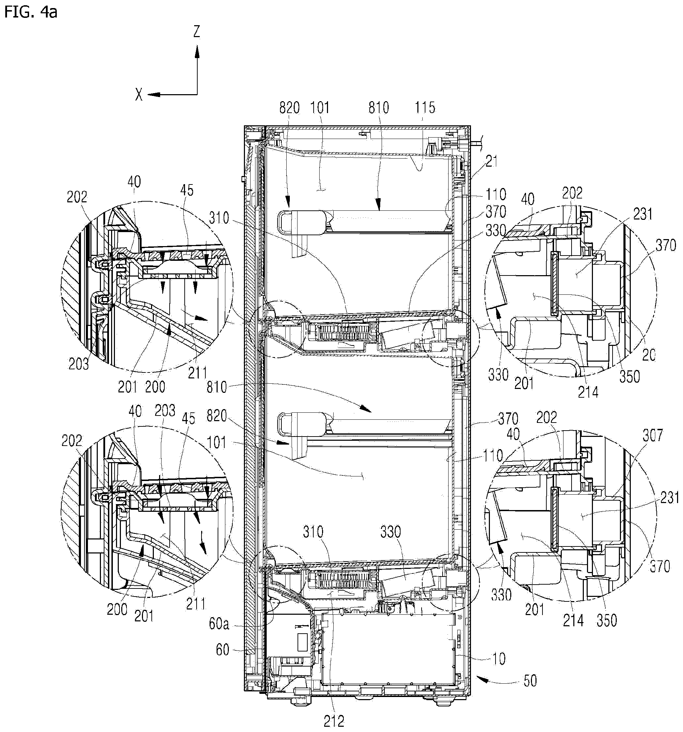

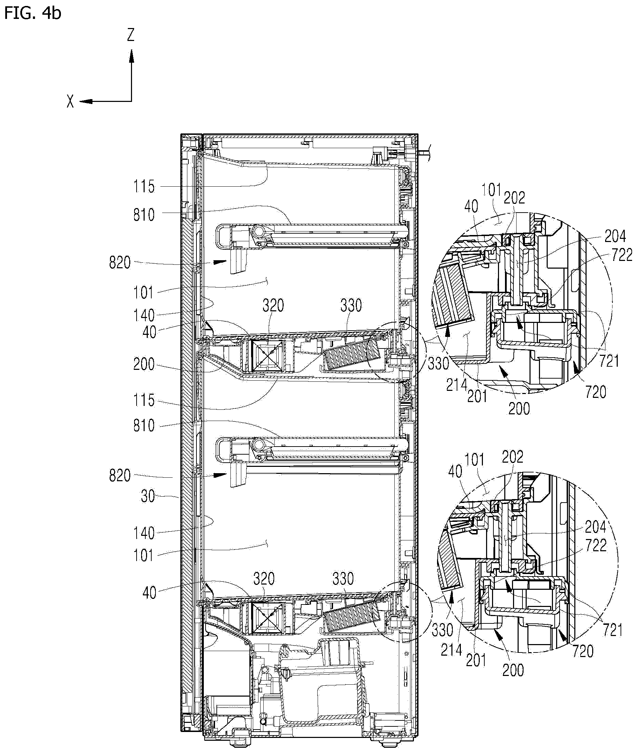

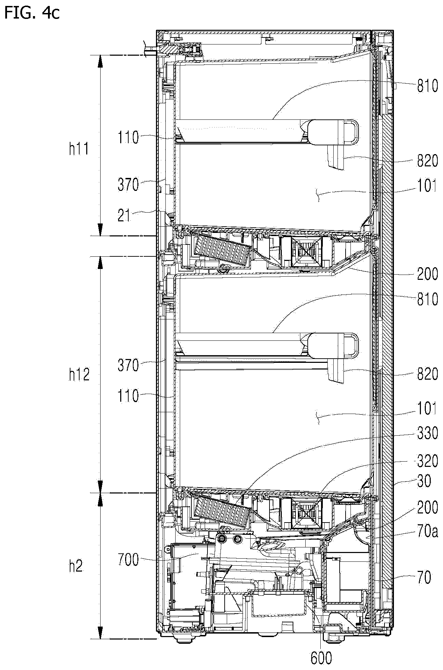

FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.

The shoes care device 1 according to one embodiment of the present invention includes management devices 2a and 2b. In the shoes care device 1, a plurality of management devices 2a and 2b may be provided. In one embodiment, the shoes care device 1 may include a first management device 2a and a second management device 2b. That is, the shoes care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include a outlet 203 and a nozzle 820.

The management devices 2a and 2b may include the module housing 200 forming a connection path F10.

The management devices 2a and 2b may include a regeneration path F20. The management devices 2a and 2b may include a heating part 320.

The management devices 2a and 2b may include a conversion flow path F10a.

The management devices 2a and 2b may include a damper 350.

The shoes care device 1 may include a steam generator 700 and a steam valve 710.

The shoes care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoes care device 1 may be provided, and various flow paths constituting the shoes care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid.

The connection path F10 forms a passage through which air and/or condensed water inside the shoes care device 1 moves.

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 45 penetrating in the vertical direction may be formed in the main shelf 40 so as to move the air.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoes care device 1 according to one embodiment of the present invention.

The inner cabinet 100, the module housing 200, and the machine room 50 may be continuously arranged from the upper side to the lower side. When the shoes care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoes care device 1.

In the shoes care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoes care device 1 inside. The water supply tank 60 may be configured to store water supplied into the steam generator 700 inside.

In order to supply water from the water supply tank 60 into the shoes care device 1, a water pump 61 may be connected to the water supply tank 60. The water supply tank 60 for moving water and the first water pump 61 may be connected by a pipe, a hose, etc.

The drain tank 70 may be configured to store water discharged from the shoes care device 1 inside. The drain tank 70 may store water condensed inside the shoes care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoes care device 1.

In order to control the controller 10, the shoes care device 1 may be provided with a storage medium in which an application program is stored, and the controller 10 may be configured to control the shoes care device 1 by driving an application program according to information input to the shoes care device 1 and the information output from the shoes care device 1.

The controller 10 may control the first management device 2a and the second management device 2b constituting the shoes care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoes care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoes care device 1, the door 30, the water supply tank 60, and the drain tank 70 are formed on the same side, and when the door 30 is opened, the water supply tank 60 and the drain tank 70 may be exposed and separated from the shoes care device 1.

In the arrangement explained above, even if left and right sides and a rear side of the shoes care device 1 are blocked by other goods or structures, the door 30 may be opened on a front side of the shoes care device 1, and the water supply tank 60 and the drain tank 70 can be separated from or coupled again to the shoes care device 1.

A control panel 33 for controlling the shoes care device 1 is provided on an outer side of the door 30. The control panel 33 may be formed of a touch screen. A control unit (controller 10) is provided in the inner space of the door 30 to control each component of the shoes care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoes care device 1 according to one embodiment of the present invention may be further provided with a dedicated door of the machine room 50 to open and close the machine room 50 separately from the door 30.

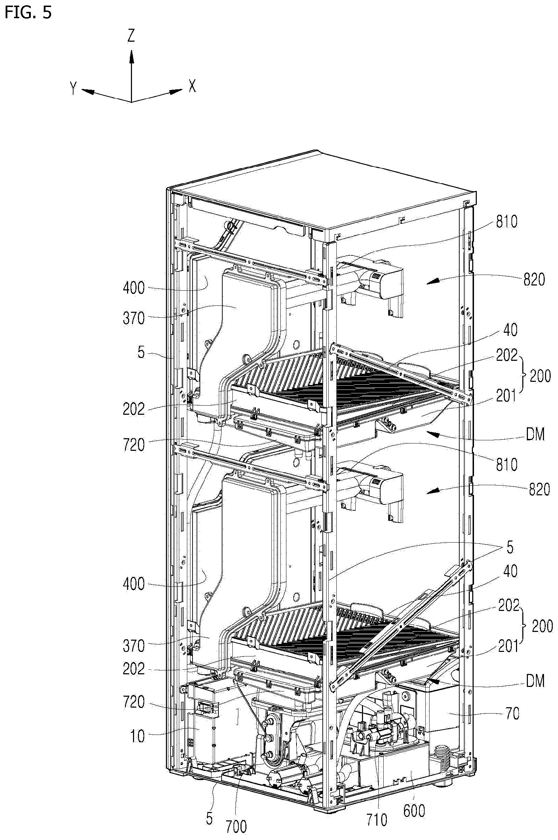

FIG. 5 is a perspective view illustrating a state in which the door 30, the outer cabinet 20, and the inner cabinet 100 are removed from the shoes care device 1 according to one embodiment of the present invention.

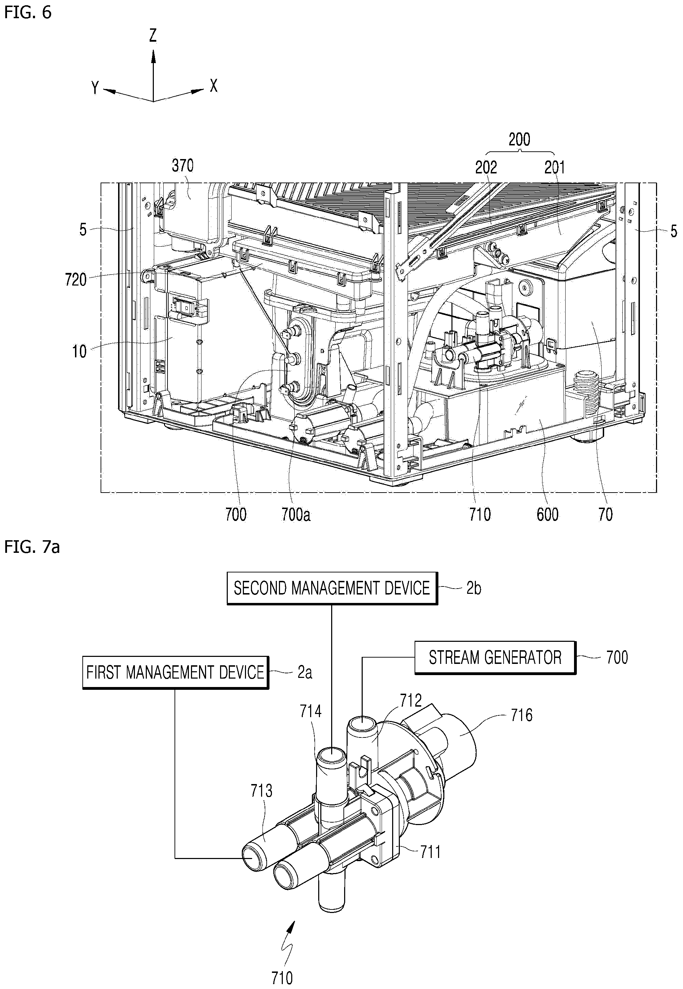

FIG. 6 is a perspective view illustrating a machine room part of the shoes care device 1 illustrated in FIG. 5.

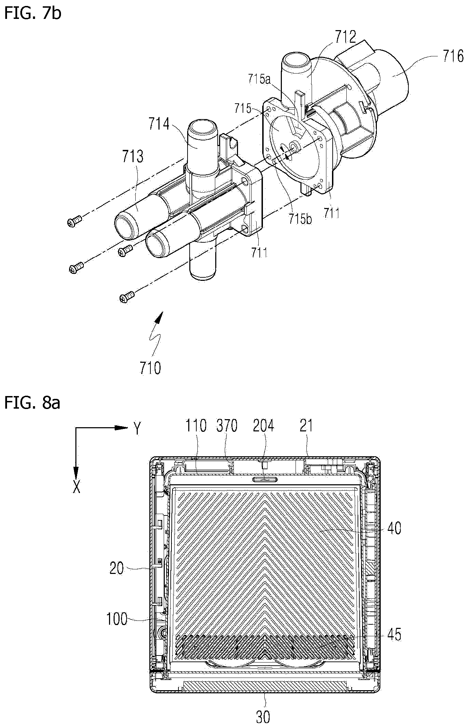

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoes care device 1 is provided with the steam generator 700 as a device configured so as to generate moisture inside the inner cabinet 100. The steam generator 700 may be provided inside the machine room 50. The steam generator 700 is configured to generate steam and selectively supply moisture and steam to the inside of the inner cabinet 100.

The shoes care device 1 according to one embodiment may be provided with one steam generator 700, and the shoes care device 1 according to another embodiment may be provided with two or more steam generators 700.

When the shoes care device 1 is provided with one steam generator 700, the steam generator 700 may be configured to supply steam into the inner cabinet 100 of the first management device 2a and/or into the inner cabinet 100 of the second management device 2b.

When the shoes care device 1, one steam generator 700 may be provided with two or more steam generators 700, one of the steam generators 700 may supply steam to the inner cabinet 100 of the first management device 2a, and the other steam generator 700 may supply steam to the inner cabinet 100 of the second management device 2b.

Moist air formed by the steam generator 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 is provided with a separate heater 700a that heats an inner space and water in the inner space and is configured to heat water to generate steam and supply the heated water to the accommodation space 101 of the inner cabinet 100.

an external faucet, etc., as a water supply source for supplying water to the steam generator 700, may be used, or a container-type water supply tank provided on one side of the machine room 50 may be used. The steam generator 700 may generate steam by receiving water from the water supply tank 60.

The water supply tank 60 for the movement of water and the steam generator 700 may be connected by a pipe, a hose, etc.

The steam generator 700 for the movement of steam and the inner cabinet 100 may be connected by a pipe, a hose, etc. In the shoes care device 1 according to one embodiment of the present invention, as described below, the steam generated by the steam generator 700 may be supplied into the inner cabinet 100 after passing through the steam valve 710 and a steam separator 720. In this case, in order to move the steam, the steam generator 700 and the steam valve 710 may be connected by a pipe, a hose, etc., and the steam valve 710 and the steam separator 720 may also be connected by a pipe, a hose, etc.

The steam valve 710 may be disposed adjacent to the steam generator 700, and the steam valve 710 may be provided in the machine room 50. The steam generator 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam generator 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716. In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam generator 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 715 is provided inside the steam valve 710 (inside the valve housing 711) and is configured to open and close a flow path inside the steam valve 710. The valve disk 715 may be configured to selectively open and close a flow path of the first valve outlet 713 and a flow path of the second valve outlet 714.

The valve disk 715 is disposed between the valve inlet 712 and the first valve outlet 713, or between the valve inlet 712 and the second valve outlet 714, inside the valve housing 711. The valve disk 715 allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 715 may be formed in a circular plate shape and may include a valve hole 715a. The valve hole 715a is a hole penetrating the valve disk 715. The valve disk 715 may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 715 to rotate the valve disk 715.

The controller 10 may control the steam valve 710 by controlling an operation of the valve motor 716.

The valve disk 715 rotates by the operation of the valve motor 716, and the valve disk 715 opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoes care device 1 according to one embodiment of the present invention, the valve disk 715 may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam generator 700 may be supplied to the accommodation space 101 of the first management device 2a, or may be supplied to the accommodation space 101 of the second management device 2b. In this case, the steam generated by the steam generator 700 may be supplied to the accommodation space 101 of the first management device 2a or the accommodation space 101 of the second management device 2b without a pressure drop, and even when only one steam generator 700 is provided in the shoes care device 1, the steam may be sufficiently and stably supplied to the accommodation spaces 101 of each of the two inner cabinets 100.

In one embodiment of the present invention, the controller 10 may control the stream valve 710 such that when the heating part 320 of the first management device 2a is turned off (when a heater 321 of the heating part 320 is turned off), the valve disk 715 closes or opens the first valve outlet 713, and when the heating part 320 of the first management device 2a is turned on (when the heater 321 of the heating part 320 is turned on), the valve disk 715 closes the first valve outlet 713.

In addition, the controller 10 may control the stream valve 170 such that when the heating part 320 of the second management device 2b is turned off (when the heater 321 of the heating part 320 is turned off), the valve disk 715 closes or opens the second valve outlet 714, and when the heating part 320 of the second management device 2b is turned on (when the heater 321 of the heating part 320 is turned on), the valve disk 715 closes the second valve outlet 714.

In the shoes care device 1 according to one embodiment of the present invention, by controlling the steam valve 710 by the controller 10, the steam generated in the steam generator 700 may be selectively or simultaneously supplied to the accommodation space 101 of the first management device 2a and the accommodation space 101 of the second management device 2b, and whether the steam is supplied or not may be controlled according to a use state of the shoes care device 1.

FIG. 8a is a cross-sectional view taken along line D-D' of the shoes care device 1 illustrated in FIG. 3.

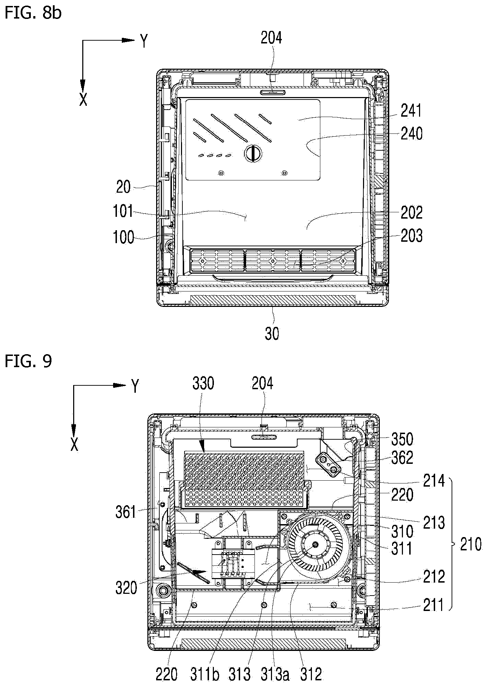

FIG. 8b is a view illustrating a state in which the main shelf 40 is removed from the shoes care device 1 illustrated in FIG. 8a.

FIG. 9 is a cross-sectional view taken along line E-E' of the shoes care device illustrated in FIG. 3.

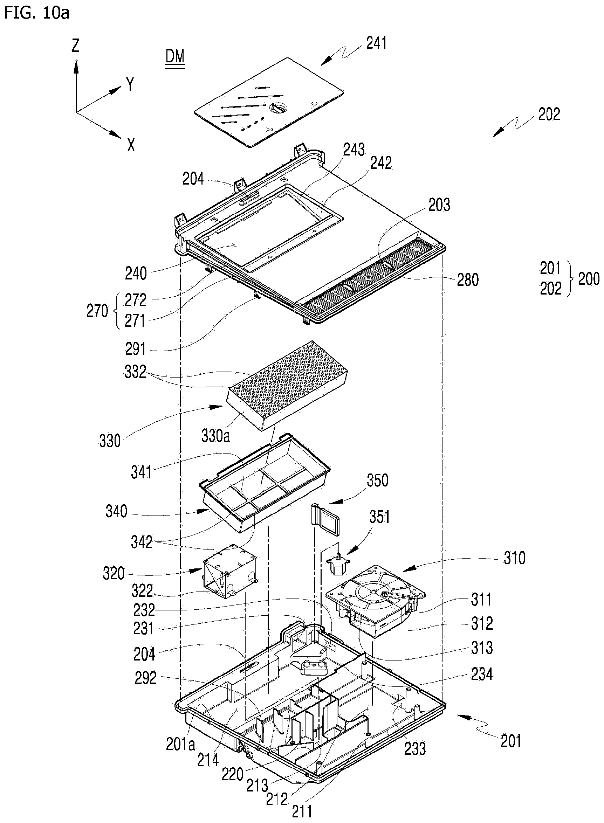

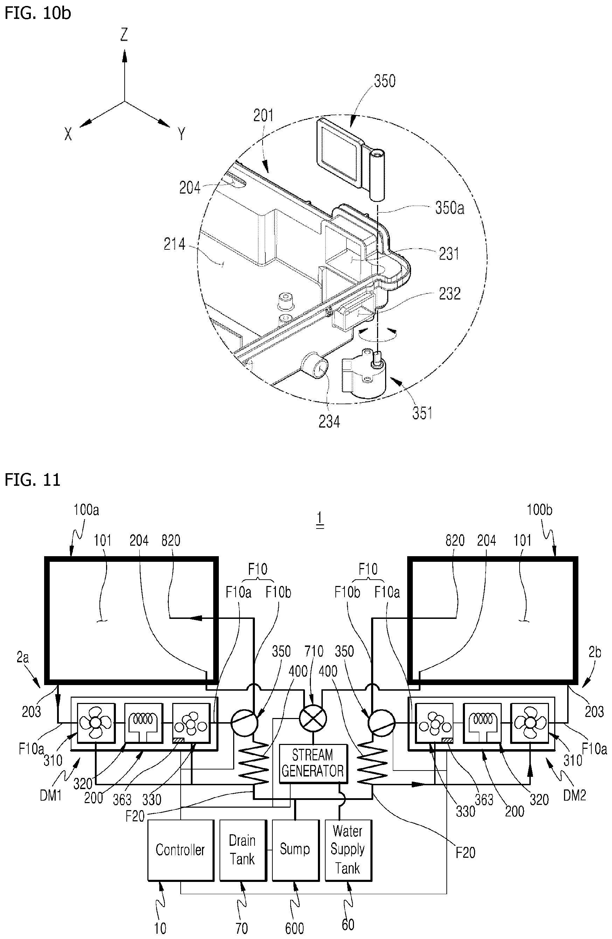

FIG. 10a is an exploded perspective view illustrating a drying module in the shoes care device 1 according to one embodiment of the present invention. FIG. 10b is an exploded perspective view illustrating a partial configuration of the drying module at a part to which the damper 350 is coupled.

In the shoes care device 1 according to one embodiment of the present invention, the dehumidifying part 330 may be used as a means that dehumidifies air.

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (A) are regularly arranged, and may function as dehumidification by adsorbing the moisture in the air.

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A blowing fan 313 may be provided inside the blowing part 310. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of p the blowing part 310 (a rotation of the blowing fan 313), and the air inside the connection path F10 may be ventilated.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoes care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed.

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoes care device 1 according to one embodiment may be provided with two sets.

Such a set may be provided in each of the first management device 2a and the second management device 2b.

Such a set may form a drying module DM in the shoes care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoes care device 1 according to one embodiment of the present invention, a plurality of drying modules DM may be provided.

In the shoes care device 1 according to one embodiment of the present invention, a pair of drying modules DM may be provided. When the shoes care device 1 is provided with the pair of drying modules DM, one of the drying modules DM may form a 'drying module A (DM1) ' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoes care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoes care device according to one embodiment of the present invention may be formed in a structure in which the dehumidifying part 330 may be detachable from the module housing 200. The structure of the shoes care device provides an advantageous advantage in maintaining and managing the dehumidifying part 330 and the shoes care device 1 on the whole.

On the other hand, the dehumidifying part 330 may be repeatedly used by regeneration, but with the repeated use, the dehumidifying part 330 needs to be replaced.

Considering these descriptions, the shoes care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoes care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoes care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoes care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoes care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user.

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

a network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoes care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoes care device 1, such moisture may regenerate bacteria or cause odors. This is why there is need for countermeasures to solve the problems, and the shoes care device 1 according to one embodiment of the present invention can effectively prevent water from leaking in consideration of such problems.

In the shoes care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoes care device 1 with excellent regeneration efficiency.

In the shoes care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoes care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoes care device 1 according to one embodiment of the present invention, steam generated by the steam generator 700 is supplied to the accommodation space 101 of the inner cabinet 100, and to this end, the shoes care device 1 includes a steam inlet 204.

The steam inlet 204 forms an inlet through which steam is supplied to the accommodation space 101 of the inner cabinet 100.

In the shoes care device according to one embodiment of the present invention, the steam inlet 204 is formed in the module housing 200.

The steam inlet 204 may be formed in a rear part of the module housing 200 based on the first direction (X direction). The steam inlet 204 may be formed to vertically penetrate the module housing 200. The steam inlet 204 may be formed to vertically penetrate the module case 201 and the module cover 202. The steam inlet 204 may be formed in the center in a right-left direction at the rear part of the module housing 200.

The steam inlet 204 may be formed on a rear edge of the module housing 200, and may be formed directly behind a position where the third module chamber 214 is formed. The third module chamber 214 and the steam inlet 204 are shielded from each other.

In the module housing 200, the module cover 202 forms the bottom surface of the accommodation space 101, and when the module housing 200 is coupled to the inner cabinet 100, the steam inlet 204 is formed behind the bottom of the inner cabinet 100.

The steam generator 700 and the steam valve 710 are disposed in a lower part, the distance from the steam generator 710 to the steam inlet 204 can be reduced by forming the module housing 200, and the steam inlet 204 in a rear part of the module housing 200, and an increase in the load required for the supply of steam can be prevented. Accordingly, steam may be smoothly supplied from the steam generator 700 to the steam inlet 204

FIG. 11 is a diagram illustrating a connection relationship between components and a flow of fluid in the shoes care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoes care device 1.

In the nozzle 820, a hole through which air is discharged is formed in the accommodation space 101 of the inner cabinet 100, and the nozzle 820 may form a last part of the connection path F10.

In the shoes care device 1 according to one embodiment of the present invention, since the nozzle 820 is configured to be movable to various positions inside the inner cabinet 100, the shoes may be managed in the various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path F10a and a drying flow path F10b. The conversion flow path F10a and the drying flow path F10b form a movement path of air sequentially connected to each other. The air in the connection path F10 may sequentially move through the conversion flow path F10a and the drying flow path F10b.

The conversion flow path F10a forms an upstream section of the connection path F10, which is connected to the outlet 203. The conversion flow path F10a may be a section in which the blowing part 310, the heating part 320, and the dehumidifying part 330 are disposed. The conversion flow path F10a may be formed by the module housing 200, and the module chamber 210 inside the module housing 200 may form the conversion flow path F10a.

The conversion flow path F10a may be a section in which humid air moves and dries. The conversion flow path F10a may be a section in which air is dehumidified by the dehumidifying part 330.

Meanwhile, the conversion flow path F10a may be a section in which the dehumidifying part 330 (the dehumidifying material 331) are regenerated.

The drying flow path F10b forms a downstream section of the connection path F10, which connects the conversion flow path F10a to the nozzle 820. A flow path formed by the drying air duct 370, the nozzle duct 810, and the nozzle 820 may form the drying flow path F10b.

The drying passage F10b may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path F10a may not communicate with each other such that the drying flow path F10b and the conversion flow path F10a block each other.

Accordingly, when air is dehumidified by the dehumidifying part 330 in the conversion flow path F10a, the dried air moves through the drying flow path F10b.

The dry air duct 370 may be fixedly coupled to an outer wall surface of the inner cabinet 100, and the nozzle duct 810 may be provided inside the inner cabinet 100.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path F10b. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path F10a, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid.

The regeneration passage F20 forms a passage through which air and/or condensed water inside the shoes care device moves.

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoes care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the shoes care device 1 according to one embodiment of the present invention, when the drying module DM operates in the regeneration mode, the regeneration path F20 communicates with the conversion flow path F10a, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoes care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoes care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path F10a and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path F10b and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path F10a of the first management device 2a may flow through the drying flow path F10b, and the air in the conversion flow path F10a of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path F10a of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoes care device 1 according to one embodiment of the present invention, the first management device 2a and the second management device 2b individually include the inner cabinet 100, the connection path F10, the blowing part 310, and the dehumidifying part 330. In addition, the shoes care device 1 includes the steam generator 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam generator 700, the steam generator 700 can be efficiently utilized in the shoes care device 1, and the shoes care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoes care device 1 can be achieved.

In the shoes care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller (10 may control the damper 350 to open the drying flow path F10b and close the regeneration path F20 when the heating part 320 is turned off, and may control the damper 350 to close the drying flow path F10b and open the regeneration path F20 when the heating part 320 is turned on.

The controller 10 may control each component of the shoes care device 1 such that air flowing into the module chamber 210 from the accommodation space 101 and passing through the dehumidifying part 330 moves along the drying flow path F10b when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 715 closes or opens the first valve outlet 713, when the heating part 320 of the first management device 2a is turned on, the valve disk 715 closes the first valve outlet 713, when the heating part 320 of the second management device 2b is turned off, the valve disk 715 closes or opens the second valve outlet 714, and when the heating part 320 of the second management device 2b is turned on, the valve disk 715 closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoes care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoes care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoes care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoes care device 1 operates to refresh the shoes.

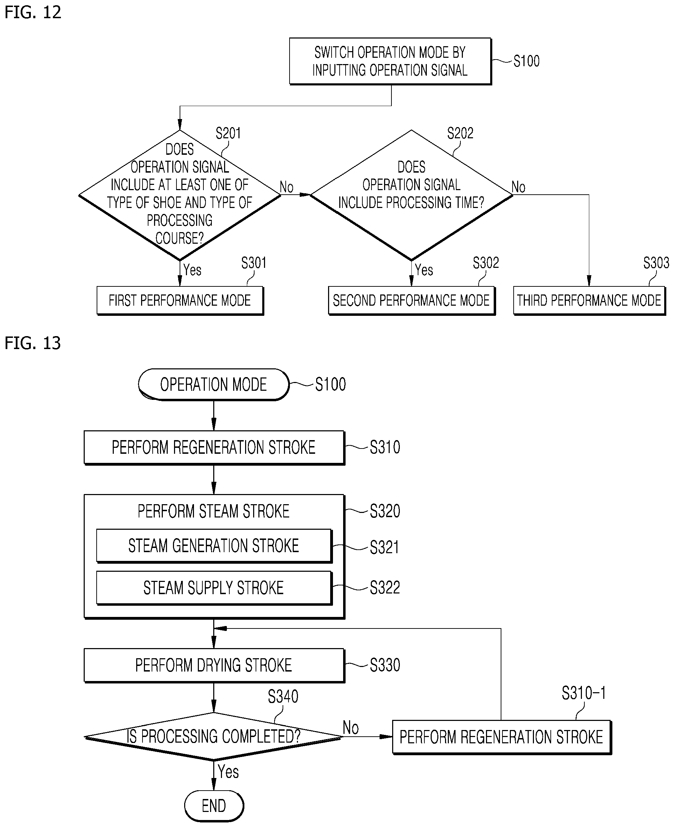

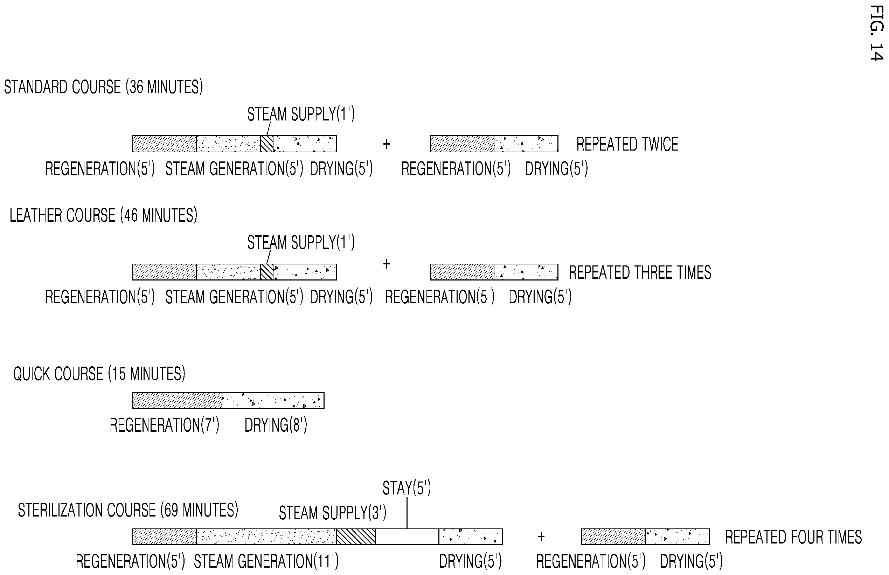

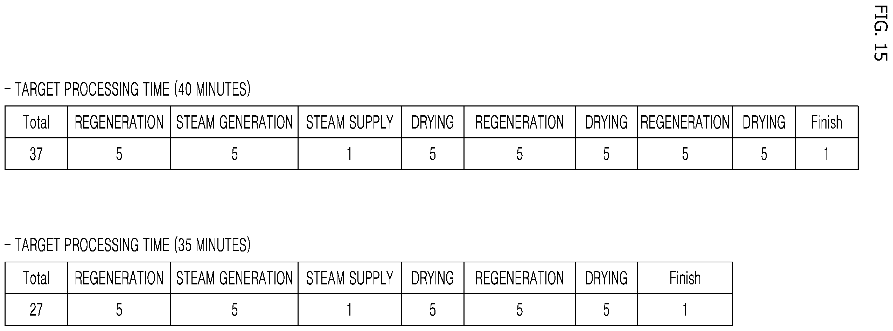

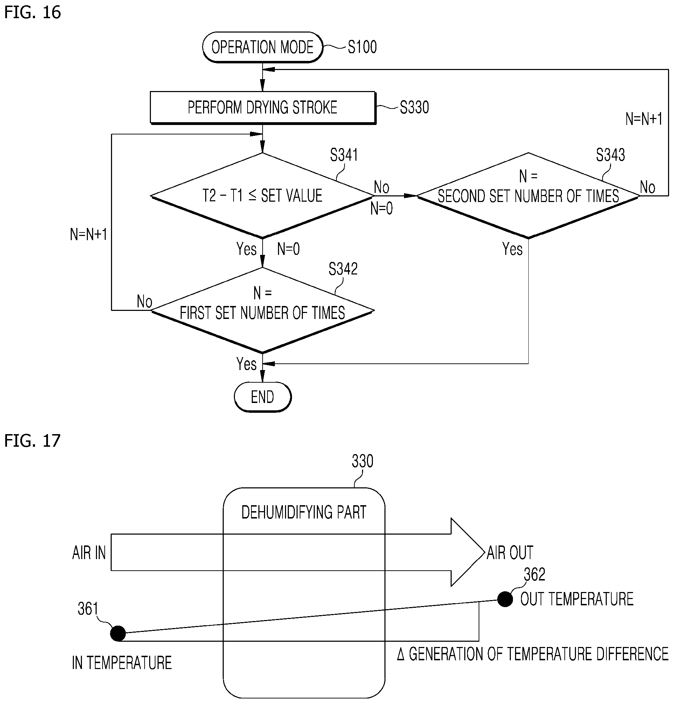

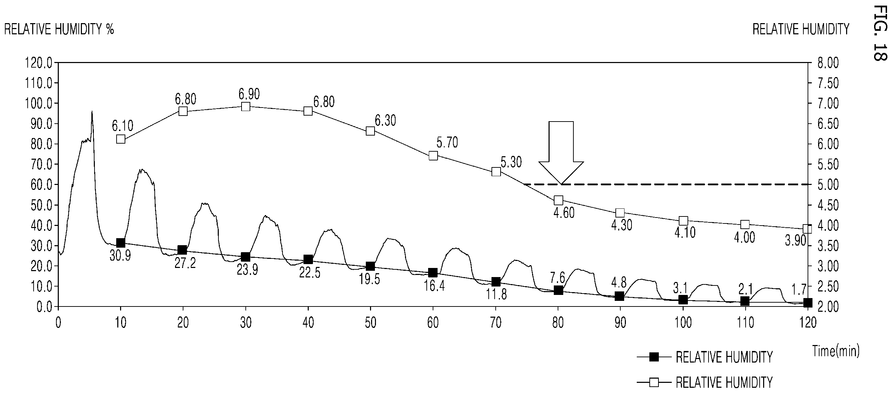

FIG. 12 is a diagram illustrating a stroke progress depending on the type of operation signal in a control method of the shoes care device 1 according to an embodiment of the present invention. FIG. 13 is a diagram illustrating the stroke progress after a current mode is switched to an operation mode in the control method of the shoes care device 1 according to an embodiment of the present invention. FIG. 14 is a diagram exemplarily illustrating a first performance mode in the control method of the shoes care device 1 according to an embodiment of the present invention. FIG. 15 is a diagram exemplarily illustrating a second performance mode in the control method of the shoes care device 1 according to an embodiment of the present invention. FIG. 16 is a diagram exemplarily illustrating a third performance mode in the control method of the shoes care device 1 according to an embodiment of the present invention. FIG. 17 is a diagram illustrating a temperature change of air passing through a dehumidifying part 330 in the shoes care device 1 according to an embodiment of the present invention. FIG. 18 is a diagram illustrating a humidity change depending on a temperature difference of the air passing through the dehumidifying part 330 in the shoes care device 1 according to an embodiment of the present invention.

According to an embodiment of the present invention, a shoes care device 1 may be configured to include an inner cabinet 100, a connection path F10, a blowing part 310, a dehumidifying part 330, a heating part 320, a regeneration path F20, a controller 10, and a control panel 33.

In this case, the controller 10 may control a regeneration stroke and a drying stroke according to the type of operation signal input into the control panel 33.

The controller 10 as a part that controls the shoes care device 1 may control a module chamber 210 so that the connection path F10 and the regeneration path F20 are selectively opened/closed according to the dehumidifying part 330 being heated.

That is, the controller 10 may selectively open/close the connection path F10 and the regeneration path F20 according to the dehumidifying part 330 being regenerated.

As a result, the controller 10 may control a drying stroke in which air is moved and dehumidified along the connection path F10 to dry an accommodation space 101 and a regeneration stroke in which the air is moved and heated along the regeneration path F20 to dry the dehumidifying part 330 to be selectively performed.

The control panel is a part into which the operation signal is inputtable by a user, and the user manipulates the control panel 330 to input the operation signal for each component of the shoes care device 1.

In this case, the control panel 33 may be configured to include a touch screen. A control unit (controller 10) linked with the control panel 33 and controlling each component of the shoes care device 1 is provided in an inner space part of the door 30. The controller 10 may be provided inside a machine room 50.

In addition, the user interface (UI) is disposed on the control panel 33, and the user manipulates the UI disposed on the control panel 33 to input enter the operation signal for each component of the shoes care device 1.

Meanwhile, when the user inputs the operation signal into the control panel 33, the user may input a specific operation signal by considering in which stroke order, and/or time to operate the shoes care device 1.

Accordingly, in that a stroke order and/or time desired by the user is reflected to the type of operation signal input into the control panel 33, the shoes care device 1 need to be operated according to such a user's intention.

As a result, the controller 10 may be preferably configured to control the regeneration stroker and the drying stroke by reflecting the user's intention through the type of operation signal input into the control panel 33.

As such, in the shoes care device 1 according to the embodiment, since the regeneration stroke and the drying stroke are made by an optimal stroke to which the user's intention is reflected according to the type of operation signal input by the user, the user may more conveniently and efficiently use the shoes care device 1.

In the shoes care device 1 according to an embodiment of the present invention, when the operation signal is input into the control panel 33, the controller 10 may control the regeneration stroke and the drying stroke to be performed at least once.

That is, if the processing for the shoes reaches an appropriate level through one regeneration and one drying stroke, the operation of the shoes care device 1 may be terminated, but otherwise, the regeneration stroke and the drying stroke may be repeatedly performed until the processing for the shoes reaches the appropriate level.

As such, in the shoes care device 1 according to the embodiment, since the regeneration stroke and the drying stroke are performed at least once in the operation mode, the stroke may be performed repeatedly until appropriate processing for the shoes is achieved.

The shoes care device 1 according to an embodiment of the present invention may further include a steam generator 700 configured to supply the steam to the inner cabinet 100.

In this case, the controller 10 may control a steam stroke of supplying the steam by the steam generator 700.

That is, the controller 10 controls the steam stroke in addition to the regeneration stroke and the drying stroke to allow the regeneration stroke, the drying stroke, and the steam stroke for processing the shoes to be appropriately performed.

As such, in the shoes care device 1 according to the embodiment, since the steam stroke is performed in the operation mode, a refresh effect by puffing of a shoes material can be shown jointly with an effect of sterilization by a high temperature of steam.

In the shoes care device 1 according to the embodiment of the present invention, when the operation signal input into the control panel 33 includes at least one of the type of shoes and the type of processing course, the controller 10 may control the regeneration stroke, the drying stroke, and the steam stroke in a first performance mode of conducting the stroke in a predetermined order to correspond to the operation signal.

Here, the order predetermined to correspond to the operation signal refers to an order of the stroke which is most appropriately set based on experimental data in a design and manufacturing process of the shoes care device 1.

For example, as illustrated in FIG. 14, when the type of shoes is not general, and special processing is required like leather, the user may input the type of shoes such as 'leather' into the control panel 33.

As a result, the shoes care device 1 may perform processing for the shoes according to the stroke predetermined in a most appropriate order when the type of shoes is 'leather'.

Further, the user may directly select the type of processing course for processing the shoes as 'standard course', 'quick course', and 'sterilization course', and input the selected type into the control panel 33.

As a result, the shoes care device 1 may perform the processing for the shoes according to the stroke predetermined in the most appropriate order in each course selected by the user.

As such, in the shoes care device 1 according to the embodiment, when the operation signal includes at least one of the type of shoes and the type of processing course, the first performance mode in which the stroke is conducted in the predetermined order, a stroke to which the user's intention to conduct the processing for the shoes in a specific order may be performed.

In the shoes care device 1 according to an embodiment of the present invention, when the operation signal input into the control panel 33 includes a processing time, the controller 10 may control the regeneration stroke, the drying stroke, and the steam stroke in a second performance mode of distributing and conducting the stroke within the processing time of the operation signal.

That is, when the user intentionally inputs a time when the processing of the shoes is terminated, the stroke may be performed so that best processing for the shoes is performed within a limit not exceeding the processing time.

For example, as illustrated in FIG. 15, when the user inputs a target processing time as the time when the processing for the shoes is terminated as 40 minutes, the shoes care device 1 may conduct the stroke within the limit not exceeding 40 minutes.

In this case, since the regeneration stroke and the drying stroke are performed at one cycle, and the steam stroke may be preferably performed between one regeneration stroke and one drying stroke, each stroke may be distributed and conducted for a total of 37 minutes within the target processing time of 40 minutes.

In addition, when the user inputs the target processing time as the time when the processing for the shoes is terminated as 35 minutes, each stroke may be distributed and conducted for a total of 27 minutes within the target processing time of 30 minutes by considering such a point.

In this case, when the regeneration stroke and the drying stroke which are one cycle are further performed, the target processing time may be exceeded, the stroke may be controlled to be conducted only up to 27 minutes.

Meanwhile, the stroke may be conducted so that a total stroke time does not exceed the target processing time as described above, but besides, the stroke may also be controlled to be conducted within a limit in which the total stroke time minimally exceeds the target processing time through a case where the user changes setting in advance.

That is, when the target processing time of FIG. 15 is 35 minutes, the regeneration stroke and the drying stroke which are one cycle are performed once more, so the stroke may be conducted up to a total of 37 minutes.

As such, in the shoes care device 1 according to the embodiment, when the operation signal includes the processing time, the second performance mode of distributing and conducting the stroke is performed within the processing time, a stroke to which the user's intention to process the shoes is reflected may be performed within a specific processing time.

In the shoes care device 1 according to an embodiment of the present invention, when the operation signal input into the control panel 33 does not include the type of shoes, the type of processing course, and the processing time, the controller 10 may control the regeneration stroke, the drying stroke, and the steam stroke in a second performance mode of conducting the stroke up to a state of being capable of estimating that the processing for the shoes is completed.

Here, the state of being capable of estimating that the processing for the shoes is completed refers to a state in which it may be judged that appropriate processing is made by sensing temperatures and/or humidities of the shoes, the accommodation space, and the connection path F10 through a separate component such various sensors.

For example, as illustrated in FIG. 16, an air temperature difference (T2 - T1) before and after passing through the dehumidifying part 330 is sensed to judge that the appropriate processing is made.

When the user just operates the shoes care device 1 without inputting a particular operation signal, the user may intend to conduct optimal processing for the shoes without regarding the stroke order or the processing time.

As a result, in the shoes care device 1, the stroke may be conducted until reaching the optimal processing state for the shoes, and then automatically terminated.

As such, in the shoes care device 1 according to the embodiment, when the operation signal does not include the type of shoes, the type of processing course, and the processing time, the third performance mode in which the stroke is conducted up to the state of being capable of estimating that the processing for the shoes is completed, so the stroke to which the user's intention to conduct the optimal processing for the shoes is reflected may be performed regardless of the order or the processing time.

In the shoes care device 1 according to an embodiment of the present invention, the controller 10 may control the regeneration stroke to be performed earlier than the drying stroke.

That is, since the regeneration stroke and the drying stroke are performed at one cycle, and the regeneration cycle is performed before the drying stroke at such a cycle, so the dehumidifying part 330 regenerated before the progress of the drying stroke may continuously perform the dehumidification function in the best state.

In the shoes care device 1 according to an embodiment of the present invention, the controller 10 may control the steam stroke to be performed between one regeneration stroke and one drying stroke.

That is, since the steam stroke is performed between one regeneration stroke and one drying stroke in the operation mode, a humidity which may be relatively high in the steam stroke is repeatedly lowered in a subsequent drying stroke to enhance the processing efficiency for the shoes.

Hereinafter, a control method of the shoes care device 1 according to an embodiment of the present invention will be described.

First, an operation signal is input by the user in a standby mode state, which is switched to an operation mode (S100). In this case, the user may input the operation signal through the control panel 33.

Next, a regeneration stroke S310 in which air is moved and heated along the regeneration path F20 to regenerate the dehumidifying part 330 and a drying stroke S330 in which the air is moved and dehumidified along the connection path F10 to dry the accommodation space 101 are performed.

In this case, steps S310 and S330 may be performed according to the type of operation signal. That is, the type of operation signal is distinguished in steps S201 and S202 illustrated in FIG. 12, and as a result, steps S310 and S330 may be conducted.

As a result, in that the stroker order and/or time desired by the user are reflected to the type of operation signal input by the user, steps S310 and S330 may be performed according to the user's intention.

In the control method of the shoes care device 1 according to an embodiment of the present invention, after being switched to step S100, steps S310 and S330 may be performed at least once.

That is, in the shoes care device 1, steps S310 and S330 are conducted once, and then when it is judged that processing for shoes reaches an appropriate level in step S340, the operation of the shoes care device 1 may be terminated.

On the contrary, when it is judged that the processing for the shoes does not reach the appropriate level, steps S310-1 and S330 may be repeatedly performed until reaching the appropriate level.

The control method of the shoes care device 1 according to an embodiment of the present invention may further include a step S320 of performing the steam stroke of supplying steam to the accommodation space 101.

That is, the shoes care device 1 conducts steps S310, S320, and S330 to appropriately perform the regeneration stroke, the drying stroke, and the steam stroker for processing the shoes.

In the control method of the shoes care device 1 according to an embodiment of the present invention, when the operation signal includes at least one of the type of shoes and the type of processing course, steps S310, S320, and S330 may be performed in a first performance mode S301 of conducting the stroke in an order predetermined to correspond to the operation signal.

That is, as illustrated in FIG. 12, when it is judged that the operation signal includes at least one of the type of shoes and the type of processing course in step S201, the first performance mode S301 is performed to reflect a user's intention to process the shoes in a specific order.

In the control method of the shoes care device 1 according to an embodiment of the present invention, when the operation signal includes the processing time, steps S310, S320, and S330 may be performed in a second performance mode S302 of distributing and conducting the stroke within the processing time of the operation signal.

That is, as illustrated in FIG. 12, when it is judged that the operation signal includes the processing time in step S202, the second performance mode S302 is performed to reflect the user's intention to process the shoes in the specific order.

In the control method of the shoes care device 1 according to an embodiment of the present invention, when the operation signal does not include at least one of the type of shoes, the type of processing course, and the processing time, steps S310, S320, and S330 may be performed in a third performance mode S303 of conducting the stroke up to a state of being capable of estimating that the processing for the shoes is completed.

That is, as illustrated in FIG. 12, when it is judged that the operation signal does not include the type of shoes, the type of processing course, and the processing time in steps S201 and S202, the third performance mode S303 is performed to reflect a user's intention to achieve the optimal processing for the shoes regardless of the order or the processing time.

In the control method of the shoes care device 1 according to an embodiment of the present invention, step S301 may be performed earlier than step S330.

That is, since step S330 is performed at one cycle, and step S310 is performed before step S330 at such one cycle, so the dehumidifying part 330 regenerated before the progress of the drying stroke may continuously perform the dehumidification function in the best state.

In the control method of the shoes care device 1 according to an embodiment of the present invention, step S320 may be performed between one step S310 and one step S330.

That is, the humidity which may be relatively high in step S320 is repeatedly lowered to enhance the processing efficiency for the shoes.

According to an embodiment of the present invention, the shoes care device 1 may be configured to include an inner cabinet 100, a connection path F10, a blowing part 310, a dehumidifying part 330, a heating part 320, a regeneration path F20, and a controller 10.

In this case, the controller 10 may control the regeneration stroker to be performed earlier than the drying stroke.

When the shoes care device 1 is in a standby mode for a long time, a large amount of moisture may be adsorbed in the dehumidifying part 330. When the drying stroke of the shoes care device 1 is immediately in such a state, a drying function through the dehumidifying part 330 may not be smoothly shown.

As a result, it may be preferable that when the shoes care device 1 is switched from the standby mode to the operation mode, the shoes care device 1 continuously performs the regeneration stroke preemptively.

Further, even when the shoes care device 1 is switched to the operation mode, and performs the drying stroke after the regeneration stroke, it may be preferable to continuously conduct the drying stroke for a long time.

The moisture in the air is collected by the dehumidifying part 330 during the drying stroke, and as the amount of the collected moisture increases, the drying function through the dehumidifying part 330 may be deteriorated.

Accordingly, it is preferable that the shoes care device 1 performs the drying stroker for a predetermined time, and then performs the regeneration stroker, and then perform performs the drying stroke.

In this case, performing the regeneration stroker after the processing for the shoes is completed may be not required in that when the shoes care device 1 is thereafter switched to the standby mode state, the performance of the dehumidifying part 330 may be deteriorated.

When all circumstances are comprehensively considered, it is preferable that the regeneration stroker and the drying stroker are achieved at one cycle so that the shoes care device 1 continuously performs the regeneration stroke preemptively, and then performs the drying stroke.

As described above, in the shoes care device 1 according to the embodiment, since the regeneration stroke and the drying stroke are performed at one cycle, and the regeneration cycle is performed before the drying stroke at such a cycle, so the dehumidifying part 330 regenerated before the progress of the drying stroke may continuously perform the dehumidification function in the best state.

The shoes care device 1 according to an embodiment of the present invention may further include the control panel 33 capable of inputting the operation signal by the user.

In this case, when the operation signal is input into the control panel 33, the controller 10 may control the regeneration stroke and the drying stroke to be performed at least once.

As such, in the shoes care device 1 according to the embodiment, since the regeneration stroke and the drying stroke are performed at least once in the operation mode, the stroke may be performed repeatedly until appropriate processing for the shoes is achieved.

The shoes care device 1 according to an embodiment of the present invention may further include a steam generator 700 configured to supply the steam to the inner cabinet 100.

In this case, the controller 10 may control a steam stroke of supplying the steam by the steam generator 700.

As such, in the shoes care device 1 according to the embodiment, since the steam stroke is performed in the operation mode, a refresh effect by puffing of a shoes material can be shown jointly with an effect of sterilization by a high temperature of steam.

In the shoes care device 1 according to an embodiment of the present invention, the controller 10 may control the steam stroke to be performed between one regeneration stroke and one drying stroke.

As such, in the shoes care device 1 according to the embodiment, since the steam stroke is performed between one regeneration stroke and one drying stroke in the operation mode, the humidity which may be relatively high in the steam stroke is repeatedly lowered in a subsequent drying stroke to enhance the processing efficiency for the shoes.

In the shoes care device 1 according to an embodiment of the present invention, the steam stroker may include a steam generation stroke of conducting temperature rising up to a setting temperature by the steam generator 700 and generating the steam, and a steam supply stroker of supplying the steam generated by reaching the setting temperature to the inner cabinet 100.

The steam generator 700 need to generate the steam by heating water in order to supply the steam to the accommodation space 101. The generation of the steam may require relatively more time than a time of supplying the steam in that temperature rising is achieved up to the setting temperature.

Accordingly, it may be preferable that the steam stroker is divided into the steam generation stroker and the steam supply stroke to sufficiently generate the steam before the steam supply stroker is conducted.

As such, in the shoes care device 1 according to the embodiment, since the steam stroker includes the steam generation stroke and the steam supply stroke, a sufficient state of steam may be generated before supplying the steam to the accommodation space 101.

In the shoes care device 1 according to an embodiment of the present invention, the controller 10 may control the steam supply stroke to be performed in a state in which the blowing part 310 is driven.

In the steam generation stroker, it is enough if only the temperature rising of the steam generator 700 is achieved, and it is not necessary to operate other components of the shoes care device 1 such as the blowing part 310. When other components of the shoes care device 1 such as the blowing part 310 are still unnecessarily operated jointly, power efficiency may be deteriorated by overload.

On the contrary, in the steam supply stroker, other components of the shoes care device 1 such as the blowing part 310 related to the processing of the shoes need to be operated jointly. That is, the circulation air current is generated even during the steam supply stroke to smoothly process the shoes.

As a result, it may be preferable that the steam stroker is divided into the steam generation stroker and the steam supply stroke to perform the steam supply stroke jointly with the driving of the blowing part 310.

As such, in the shoes care device 1 according to the embodiment, since the steam supply stroke is performed in the state in which the blowing part 310 is driven, the circulation air current may be smoothly generated while the steam is supplied.

In the shoes care device 1 according to an embodiment of the present invention, the controller 10 may control the steam generation stroke to be performed in the state in which one regeneration stroke is being performed.

As described above, when other components of the shoes care device 1 are operated jointly, the power efficiency may be deteriorated by the overload.

However, conducting the steam generation stroke requiring relatively more time alone may increase an overall processing time for the shoes.

As a result, it may be preferable that the steam generation stroke is conducted jointly with other strokes within a limit not to deteriorate the power efficiency.

Meanwhile, as such, it may be preferable that the steam stroke is performed between one regeneration stroke and one drying stroke. In this case, when the steam generation stroke is performed jointly with one drying stroke, it may be inappropriate in that the steam supply stroke should be conducted after the termination of the drying stroke.

As a result, it may be preferable that the steam generation stroke is performed jointly with one regeneration stroke.

As such, in the shoes care device 1 according to the embodiment, since the steam generation stroke is performed in the state in which one regeneration stroke is being performed, a time required for the stroke before substantial shoes processing is not yet started may be minimized.

Hereinafter, a control method of the shoes care device 1 according to an embodiment of the present invention will be described.

First, the standby mode state is switched to the operation mode (S100). That is, the shoes care device 1 in the standby mode state is switched to the operation mode, so the stroke for processing the shoes may be started.

Next, a regeneration stroke S310 in which air is moved and heated along the regeneration path F20 to regenerate the dehumidifying part 330 and a drying stroke S330 in which the air is moved and dehumidified along the connection path F10 to dry the accommodation space 101 are performed.

In this case, step S310 may be performed earlier than step S330. That is, the regeneration stroke and the drying stroke may be made at one cycle so that the shoes care device 1 continuously performs the regeneration stroke preemptively, and then performs the drying stroke.

In the control method of the shoes care device 1 according to an embodiment of the present invention, step S100 may be performed by inputting the operation signal by the user. In this case, the user may input the operation signal through the control panel 33.

In addition, after being switched to step S100, steps S310 and S330 may be performed at least once.

That is, when it is judged that the processing for the shoes reaches the appropriate level, steps S310 and S330 may be conducted once or steps S310-1 and S330 may be repeatedly performed.

The control method of the shoes care device 1 according to an embodiment of the present invention may further include a step S320 of performing the steam stroke of supplying steam to the accommodation space 101.

That is, the shoes care device 1 conducts steps S310, S320, and S330 to appropriately perform the regeneration stroke, the drying stroke, and the steam stroker for processing the shoes.

In the control method of the shoes care device 1 according to an embodiment of the present invention, step S320 may be performed between one step S310 and one step S330.

That is, the humidity which may be relatively high in step S320 is repeatedly lowered to enhance the processing efficiency for the shoes.

In the control method of the shoes care device 1 according to an embodiment of the present invention, step S320 may include a step S321 in which the steam generation stroke of conducting the temperature rising up to the setting temperature and generating the steam by the steam generator 700 and a step S322 in which the steam supply stroke of supplying the steam generated by reaching the setting temperature to the accommodation space 101.

That is, the steam stroke is divided into the steam generation stroke and the steam supply stroke to sufficiently generate the steam before the steam supply stroke is conducted.

In the control method of the shoes care device 1 according to an embodiment of the present invention, step S322 may be performed in the state in which the blowing part 310 is driven.

That is, the circulation air current is generated by driving the blowing part 310 during the steam supply stroke to smoothly process the shoes.

In the control method of the shoes care device 1 according to an embodiment of the present invention, step S321 may be performed during step S310 in which one regeneration stroke is performed.

That is, step S321 is conducted jointly with step S310 within the limit not to deteriorate the power efficiency to minimize the overall processing time for the shoes.

According to an embodiment of the present invention, the shoes care device 1 may be configured to include the inner cabinet 100, the connection path F10, the blowing part 310, the dehumidifying part 330, the controller 10, a first sensor 361, and a second sensor 362.

In this case, the controller may control the drying stroke to be performed up to a state in which it may be estimated that the processing for the shoes is completed through a temperature difference of air measured by the first sensor 361 and the second sensor 362 during the performing of the drying stroke.

Specifically, the controller 10 as a part that controls the shoes care device 1 may control the drying stroke in which the air is moved and dehumidified along the connection path F10 to dry the accommodation space 101.

The first sensor 361 as a part that is disposed in a part in which the air flows into the dehumidifying part 330 in the connection path F10 to measure the temperature of the air may measure a temperature the air before passing through the dehumidifying part 330 (see FIG. 9).

The second sensor 362 as a part in which the air is discharged from the dehumidifying part 330 in the connection path F10 to measure the temperature of the air may measure the temperature of the air after passing through the dehumidifying part 330 (see FIG. 9).

As illustrated in FIG. 17, as the humidity in the air passing through a dehumidification agent 331 (in particular, zeolite) of the dehumidifying part 330 is the higher, the temperature rising of the air due to heat dissipation of the dehumidification agent 331 may be achieved.

As a result, it may be estimated that a temperature difference between the air which flows into the dehumidifying part 330 and the air discharged from the dehumidifying part 330 is the larger, the humidity in the air is the higher. On the contrary, the drying stroke is appropriately conducted, and as a result, as the humidity in the air is the lower, the temperature difference between the air which flows into the dehumidifying part 330 and the air discharged from the dehumidifying part 330 may become the smaller.

Accordingly, when the temperature difference between the air which flows into the dehumidifying part 330 and the air discharged from the dehumidifying part 330 is measured through the first sensor 361 and the second sensor 362, it may be determined whether the state in which it may be estimated that the processing for the shoes is completed is reached.

In addition, the controller 10 may control the number of performance times and/or a performance hour of the drying stroke until the processing for the shoes reaches the appropriate level based on a measurement result of the temperature difference.

As such, in the shoes care device 1 according to the embodiment, since the drying stroke is performed up to the state in which it may be estimated that the processing for the shoes is completed through the temperature difference between the air which flows into the dehumidifying part 330 and the air discharged from the dehumidifying part 330, the operation of the shoes care device 1 may be automatically terminated without a separate manipulation of the user.

In the shoes care device 1 according to an embodiment of the present invention, when the temperature difference is sensed as a set value or less, the controller 10 may control the drying stroke to be terminated by estimating that the processing for the shoes is completed.

In this case, the set value refers to any temperature difference corresponding to a relative humidity which may be handled as the processing for the shoes reaching the appropriate level through experimental data in advance.

For example, as illustrated in FIG. 18, when the drying stroke is performed, the relative humidity may be lowered. In this case, when it is handled that a case where the relative humidity is 10% or less is handled as the processing for the shoes reaching the appropriate level, a set value of a temperature difference corresponding thereto may be 5°C.

Therefore, when a temperature difference of the airs measured by the first sensor 361 and the second sensor 362, respectively is equal to or less than 5°C which is the set value, it is estimated that the processing for the shoes is completed to terminate the drying stroke.

As such, in the shoes care device 1, when the temperature difference is equal to or less than the set value, , the drying stroke is terminated, so an operation termination time of the shoes care device 1 may be more clearly determined.

The shoes care device 1 according to an embodiment of the present invention may further include the control panel 33 capable of inputting the operation signal by the user.

In this case, when the operation signal is input into the control panel 33, the controller 10 may control the regeneration stroke and the drying stroke to be performed at least once.

As such, in the shoes care device 1 according to the embodiment, since the regeneration stroke and the drying stroke are performed at least once in the operation mode, the stroke may be performed repeatedly until appropriate processing for the shoes is achieved.

In the shoes care device 1 according to an embodiment of the present invention, when it is continuously sensed at a first set number of times that the temperature difference is equal to or less than the set value, the controller 10 may control the drying stroke to be terminated by estimating that the processing for the shoes is completed.

As described above, when the temperature difference of the airs measured by the first sensor 361 and the second sensor 362, respectively is equal to or less than the set value, the drying stroke may be immediately terminated, but when the temperature is measured due to a measurement error of the first sensor 361 and the second sensor 362 or in a momentary special situation, the reliability may be deteriorated.

Accordingly, instead of immediately terminating the drying stroke when the temperature difference of the airs measured by the first sensor 361 and the second sensor 362, respectively is equal to or less than the set value, it may be preferable that the drying stroke is at last terminated when a case where the temperature difference is equal to or less than the set value is sensed continuously at a first set number of times or more.

In this case, the first set number of times may be appropriately set (for example, three times) through the experimental data in advance, and appropriately changed by considering an operating environment of the shoes care device 1 and user's tendency.

As such, in the shoes care device 1, when the temperature difference is equal to or less than the set value at the first set number of times or more, the drying stroke is terminated, so the reliability for whether the processing for the shoes being completed may be secured.

In the shoes care device 1 according to an embodiment of the present invention, when the number of sensing times of the temperature difference reaches a second set number of times, the controller 10 may control the drying stroke to be terminated by estimating that the processing for the shoes is completed.

As described above, when it is judged that the processing for the shoes reaches the appropriate level through the temperature difference of the airs measured by the first sensor 361 and the second sensor 362, respectively, the drying stroke may be terminated, but a case where the temperature difference does not decrease to the set value or less due to the measurement error of the first sensor 361 and the second sensor 362 or damage may occur.

In this case, it may be inefficient to continuously repeat the drying process because the processing time for the shoes is excessively longer.

Accordingly, regardless of the case where the temperature difference of the airs measured by the first sensor 361 and the second sensor 362, respectively is equal to or less than the set value, it may be preferable that an additional drying stroke is not conducted but terminated when the number of sensing times of the temperature difference reaches the second set number of times.

In this case, the second set number of times may be appropriately set (for example, 15 times) through the experimental data in advance, and appropriately changed by considering an operating environment of the shoes care device 1 and user's tendency.

As such, in the shoes care device 1 according to the embodiment, when the temperature difference is sensed by the second set number of times, the drying stroke is terminated, so it is possible to prevent the stroke for processing the shoes from being conducted excessively much.

In the shoes care device 1 according to an embodiment of the present invention, when the drying stroke is temporarily stopped, and then reperformed, the controller 10 may control the first set number of times and the second set number of times not to be initialized.

.A case where the stroke is momentarily stopped due to the manipulation of the user or occurrence of an unexpected situation during the operation of the shoes care device 1 may occur.

When the stroke is momentarily stopped, and then the stroke is reperformed again, if the first set number of times and the second set number of times are initialized, the number of times of the drying stroke which is conducted for the processing of the shoes may be excessively increased.

In particular, in that a predetermined part of the processing for the shoes is conducted before momentary stop, and the processing for the shoes is reperformed, and the remaining steps of the processing for the shoes is continuously conducted, it may be not preferable that the first set number of times and the second set number of times are initialized.

As such, in the shoes care device 1 according to the embodiment, when the drying stroke is momentarily stopped, and then reperformed, the stroke is not initialized and the stroke is performed in connection with the stroke before the momentary stop, so the processing for the shoes is prevented from being excessively conducted, thereby minimizing the damage to the shoes.

The shoes care device 1 according to an embodiment of the present invention may further include a heating part 320 and a regeneration path F320.

In this case, the controller 10 may control the regeneration stroke in which the air is moved and heated along the regeneration path F20 to regenerate the dehumidifying part 330, and the drying stroke to be selectively performed.

In the shoes care device 1 according to an embodiment of the present invention, the controller 10 may control the regeneration stroke to be performed earlier than the drying stroke.

That is, since the regeneration stroke and the drying stroke are performed at one cycle, and the regeneration cycle is performed before the drying stroke at such a cycle, so the dehumidifying part 330 regenerated before the progress of the drying stroke may continuously perform the dehumidification function in the best state.

The shoes care device 1 according to an embodiment of the present invention may further include a steam generator 700 configured to supply the steam to the inner cabinet 100.

In this case, the controller 10 may control a steam stroke of supplying the steam by the steam generator 700.

As such, in the shoes care device 1 according to the embodiment, since the steam stroke is performed in the operation mode, a refresh effect by puffing of a shoes material can be shown jointly with an effect of sterilization by a high temperature of steam.

In the shoes care device 1 according to an embodiment of the present invention, the controller 10 may control the steam stroke to be performed between one regeneration stroke and one drying stroke.

As such, in the shoes care device 1 according to the embodiment, since the steam stroke is performed between one regeneration stroke and one drying stroke in the operation mode, the humidity which may be relatively high in the steam stroke is repeatedly lowered in a subsequent drying stroke to enhance the processing efficiency for the shoes.

The shoes care device 1 according to an embodiment of the present invention is coupled to a lower side of the inner cabinet 100 and has the module chamber 210 which is in communication with the accommodation space 101, and the module chamber 310 may further include may further include a module housing 200 including a first module chamber 212, a second module chamber 213, and a third module chamber 214.

In this case, the blowing part 310 is accommodated in the first module chamber 212 to blow the air of the module chamber 210, the heating part 320 is accommodated in the second module chamber 213 to heat the air of the module chamber 210, the dehumidifying part 330 is accommodated in the third module chamber 214 to dehumidify the air of the module chamber 210, and the first module chamber 212, the second module chamber 213, and the third module chamber 214 may be formed at different locations from each other on the plan view.

As such, since the shoes care device 1 according to the embodiment includes the first module number 212, the second module chamber 213, and the third module chamber 214 formed at different locations on the plan view and accommodating the blowing part 310, the heating part 320, and the dehumidifying part 330, respectively, a layout of each component for performing the regeneration stroke and the drying stroke may be optimized.

In the shoes care device 1 according to an embodiment of the present invention, the air may be configured to move in the first module chamber 212, the second module chamber 213, and the third module chamber 214 in sequence.

That is, since the air is configured to move in the first module chamber 212, the second module chamber 213, and the third module chamber 214 in sequence, the drying efficiency by the dehumidifying part 330 may be enhanced by connecting the third module chamber 214 and a drying flow path at a shortest distance, and when the dehumidifying part 330 is regenerated, the air heated by the heating part 320 directly moves to the dehumidifying part 330, so the regeneration efficiency of the dehumidifying part 330 may also be enhanced.

In the shoes care device 1 according to an embodiment of the present invention, the first sensor 361 is installed in the second module chamber 213 to measure the temperature of the air which flows into the dehumidifying part 330, and the second sensor 362 is installed in the third module chamber 214 to measure the temperature of the air discharged from the dehumidifying part 330.

That is, since the second module chamber 213 measures the temperature of the air which flows into the dehumidifying part 330, and the third module chamber 214 measures the temperature of the air discharged from the dehumidifying part 330, a layout of the first sensor 361 and the second sensor 362 for measuring the temperature of the air may be effectively achieved.

Referring to FIG. 16, hereinafter, a control method of the shoes care device 1 according to an embodiment of the present invention will be described.

First, the standby mode state is switched to the operation mode (S100). That is, the shoes care device 1 in the standby mode state is switched to the operation mode, so the stroke for processing the shoes may be started.

Next, a drying stroke S330 is performed in which the air is moved and dehumidified along the connection path F10 to dry the accommodation space 101.

Next, in step S341, an air temperature difference (T2 - T1) before and after passing through the dehumidifying part 330 is sensed to judge that appropriate processing is achieved. That is, it is judged whether the air temperature difference (T2 - T1) is equal to or less than a set value to judge whether processing for shoes reaches an appropriate level.

If the air temperature difference (T2 - T1) is equal to or less than the set value, it is judged whether such a state continuously reaches a first set number of times in step S342. As a result, when such a state reaches the first set number of times, it is judged that the processing for the shoes is completed to terminate the drying stroke.

On the contrary, if the air temperature difference (T2 - T1) is equal to or less than the set value, the drying stroke may be repeatedly performed. In this case, in step S343, it is judged whether the number of sensing times of the air temperature difference (T2 - T1) reaches a second set number of times. As a result, when the number of sensing times of the air temperature difference (T2 - T1) reaches the second set number of times, the drying stroke may be terminated in order to prevent the drying stroke from being performed excessively a lot.

Hereinabove, a specific embodiment of the present invention is described and illustrated, but the present invention is not limited to the disclosed embodiment, and it may be appreciated by those skilled in the art that the embodiment can be variously modified and transformed to another specific embodiment without departing from the spirit and the scope of the present invention. Therefore, the scope of the present invention will not be defined by the described embodiment, but defined by the technical spirit disclosed in the claims.

### INDUSTRIAL APPLICABILITY

According to at least one of the embodiments of the present invention, a dehumidifying part is disposed in a module chamber to collect moisture and bacteria in the blown air, and the dehumidifying part is heated and regenerated in the module chamber, so the performance for shoes processing can be continuously appropriately maintained.

In addition, according to at least one of the embodiments of the present invention, since a connection path through which air is circulated is formed between a outlet and a nozzle disposed inside an inner cabinet, respectively, the air used for dehumidifying and deodorizing the shoes can be prevented from being exposed to a user.

In addition, according to at least one of the embodiments of the present invention, since the drying stroke is performed until the state in which completion of processing of the shoes is able to be estimated through a temperature difference between the air introduced into the dehumidifying part and the air discharged from the dehumidifying par. Therefore, the operation of the shoe care device can be automatically terminated without the user's additional operation.

In addition, according to at least one of the embodiments of the present invention, when the temperature difference is at or below the set value, the drying stroke is terminated. Therefore, the termination point of operation of the shoe care device can be determined more clearly.

In addition, according to at least one of the embodiments of the present invention, the regeneration stroke and the drying stroke are performed at least once in the operating mode. Therefore, the strokes can be repeatedly performed until the shoes are properly processed.

In addition, according to at least one of the embodiments of the present invention, when the temperature difference is continuously at or below the set value the first set number of times or more, the drying cycle is terminated. Therefore, it is possible to ensuring reliability as to whether the processing of the shoes is completed.

In addition, according to at least one of the embodiments of the present invention, when the temperature difference is detected as much as the second set number of times, the drying stroke is terminated. Therefore, it is possible to prevent the strokes for processing the shoes from be performed excessively.

In addition, according to at least one of the embodiments of the present disclosure, when the drying stroke is re-performed after a temporary stop, the stroke is not initialized and is performed continuously to the stroke performed before the temporary stop. Therefore, it is possible to prevent excessive processing of the shoes and to minimize damage to the shoes.

In addition, according to at least one of the embodiments of the present invention, the regeneration stroke and the drying stroke are performed in one cycle, and in this cycle, the regeneration stroke is performed prior to the drying stroke. Therefore, the dehumidifying part, which is regenerated before the drying stroke, can always perform the dehumidifying function in the best condition.

In addition, according to at least one of the embodiments of the present invention, the steam stroke is performed in the operating mode. Therefore, it is possible to exert a sterilization effect by high temperature of steam and a refreshing effect by swelling of the shoe material.

In addition, according to at least one of the embodiments of the present invention, in the operating mode, the steam stroke is performed between the first regeneration stroke and the first drying stroke. Therefore, it is possible to improve processing efficiency for shoes by repeatedly lowering the humidity, which may be relatively high in the steam stroke, in the subsequent drying stroke.

In addition, according to at least one of the embodiments of the present invention, the first module chamber, the second module chamber, and the third module chamber are disposed at different positions in a plan view to accommodate the blowing part, the heating part, and the dehumidifying part, respectively. Therefore, the arrangement of respective components for performing the regeneration stroke and the drying stroke process can be optimized.

In addition, according to at least one of the embodiments of the present invention, air is allowed to move sequentially through the first module chamber, the second module chamber, and the third module chamber. Therefore, since the third module chamber and the drying path are connected in the shortest distance, the drying efficiency by the dehumidifying part can be improved. Since the air heated by the heating part moves directly to the dehumidifying part at the time of regenerating the dehumidifying part, the regeneration efficiency of the dehumidifying part can also be improved.

In addition, according to at least one of the embodiments of the present invention, the temperature of the air introduced into the dehumidifying part is measured in the second module chamber, and the temperature of the air discharged from the dehumidifying part is measured in the third module chamber. Therefore, the first and second sensors configured measuring the temperature of air can be effectively arranged.

## Claims

1. A shoes care device including an inner cabinet having an accommodation space configured to accommodate shoes, comprising:
a connection path configured to provide a flow path through which air from the accommodation space is introduced and then discharged back into the accommodation space;
a blowing part disposed in the connection path and configured to blow air;
a dehumidifying part disposed in the connection path and configured to dehumidify the air;
a controller configured to control a drying stroke in which air moves along the connection path and is dehumidified to dry the accommodation space;
a first sensor disposed in a portion of the connection path through which air is introduced into the dehumidifying part and configured to measure a temperature of the air; and
a second sensor disposed in a portion of the connection path through which the air is discharged from the dehumidifying part and configured to measure a temperature of the air,
wherein the controller is configured to control the drying stroke to be performed until a state in which completion of processing of the shoes is able to be estimated through a difference of the temperatures of air measured by the first sensor and the second sensor during the drying stroke.

2. The shoe care device of claim 1, wherein the controller is configured to estimate that the processing of the shoes has been completed and control the drying stroke to be terminated when the difference of the temperatures is detected to be at or below a set value.

3. The shoe care device of claim 2, further comprising:
a control panel configured to allow a user to input an operating signal thereto,
wherein the controller is configured to control the drying stroke to be performed at least once when the operation signal is input to the control panel.

4. The shoe care device of claim 3, wherein the controller is configured to estimate that the processing of the shoes has been completed and control the drying stroke to be terminated when the difference of the temperatures is continuously detected to be at or below the set value a first set number of times.

5. The shoe care device of claim 4, wherein the controller is configured to estimate that the processing of the shoes has been completed and control the drying stroke to be terminated when a number of detections of the difference of the temperatures reaches a second set number of times.

6. The shoe care device of claim 5, wherein the controller is configured to control the first set number of times and the second set number of times not to be initialized when the drying stroke is re-performed after a temporary stop.

7. The shoe care device of claim 3, further comprising:
a heating part disposed in the connection path and configured to heat air; and
a regeneration path through which the air heated by the heating part moves and which branches from the connection path,
wherein the controller is configured to control a regeneration stroke, in which air moves along the regeneration path and is heated to regenerate the dehumidifying part, and the drying stroke to be selectively performed.

8. The shoe care device of claim 7, wherein the controller is configured to control the regeneration stroke to be performed before the drying process.

9. The shoe care device of claim 7, further comprising:
a steam generator configured to supply steam to the inner cabinet,
wherein the controller is configured to control a steam stroke in which steam is supplied by the steam generator.

10. The shoe care device of claim 9, wherein the controller is configured to control the steam stroke to be performed between the regeneration stroke at first time and the drying stroke at first time.

11. The shoe care device of claim 7, further comprising:
a module housing coupled to a lower side of the inner cabinet and provided therein with a module chamber in communication with the accommodation, wherein the module chamber comprises a first module chamber, a second module chamber, and a third module chamber,
wherein the blowing part is accommodated in the first module chamber and configured to blow air in the module chamber,
wherein the heating part is accommodated in the second module chamber and configured to heat the air in the module chamber,
wherein the dehumidifying part is accommodated in the third module chamber and configured to dehumidify the air in the module chamber, and
wherein the first module chamber, the second module chamber, and the third module chamber are provided at different positions in a plan view.

12. The shoe care device of claim 11, wherein the air in the module chamber moves sequentially through the first module chamber, the second module chamber, and the third module chamber.

13. The shoe care device of claim 12, wherein the first sensor is installed in the second module chamber and configured to measure a temperature of air introduced into the dehumidifying part, and
wherein the second sensor is installed in the third module chamber and configured to measure a temperature of air discharged from the dehumidifying part.
